Europäisches Patentamt

European Patent Office

Office européen des brevets

(19)

(11) Numéro de publication: **0 389 380**
**A1**

(12) **DEMANDE DE BREVET EUROPEEN**

(21) Numéro de dépôt: **90400804.2**

(22) Date de dépôt: **23.03.90**

(51) Int. Cl.5: **G01N 33/577, G01N 33/543**

(30) Priorité: **24.03.89 FR 8903942**

(43) Date de publication de la demande:
**26.09.90 Bulletin 90/39**

(84) Etats contractants désignés:
**AT BE CH DE DK ES FR GB GR IT LI LU NL SE**

(71) Demandeur: **SANOFI**
**40, Avenue George V**
**F-75008 Paris(FR)**

(72) Inventeur: **Carriere, Dominique**
**29 Lotissement La Coline**
**F-34160 Castries(FR)**

(74) Mandataire: **Gillard, Marie-Louise et al**
**Cabinet Beau de Loménie 55, Rue**
**d'Amsterdam**
**F-75008 Paris(FR)**

(54) **Trousse et méthode de dosage applicables à des cellules entières.**

(57) La présente invention a pour objet une trousse pour le dosage des récepteurs de surface caractéristiques d'une population ou d'une sous-population cellulaire comprenant, comme composants :

a) un support solide sur lequel sont fixés par liaison covalente ou par adsorption physique un ou plusieurs anticorps monoclonaux dirigés contre des antigènes de surface de la population cellulaire a doser ;

b) une solution contenant le ligand spécifique du récepteur membranaire de la population ou sous-population cellulaire à doser, marqué par une sonde radio-isotopique ;

Elle concerne également un procédé de dosage des récepteurs de surface d'une population ou sous-population cellulaire.

La trousse de dosage et le procédé selon l'invention peuvent être utilisés pour le dosage de la sous-population de lymphocytes T activés et portant le récepteur à l'interleukine 2.

EP 0 389 380 A1

## Trousse et méthode de dosage applicables à des cellules entières.

La présente invention concerne une trousse et une méthode de dosage utilisant cette trousse, pour le dosage de récepteurs membranaires caractéristiques de populations ou de sous-populations de cellules. La méthode de dosage et la trousse correspondante sont également destinées au dosage des cellules elles-mêmes par l'intermédiaire du dosage de leurs récepteurs de surface. Ces dosages sont applicables au diagnostic.

Les récepteurs de surface ou récepteurs membranaires font partie des marqueurs de surface cellulaires, au même titre que les antigènes de surface. Un récepteur membranaire d'une cellule est une structure macromoléculaire insérée dans la membrane d'une cellule et capable de se lier à un ligand spécifique caractéristique de ce récepteur. Un récepteur membranaire peut aussi être considéré comme un antigène et, comme tel, il peut se lier à un anticorps spécifique.

Les récepteurs membranaires d'une cellule sont spécifiques d'une population cellulaire soit parce qu'ils sont présents pour cette population dans des conditions normales de vie de la cellule, soit, parce qu'ils ont été exprimés à la surface de la cellule par l'intervention d'un facteur endogène ou exogène et marquent donc soit un état de différenciation, soit un état physiologique de la cellule considérée.

La connaissance des antigènes ou marqueurs de la surface cellulaire a fait d'énormes progrès avec la mise au point de l'hybridation lymphocytaire et la découverte des anticorps monoclonaux par KOEHLER et MILSTEIN, (Nature, 1975, *256* , 495-497). Les anticorps monoclonaux ont notamment permis la mise en évidence et l'analyse de marqueurs de surface ou antigènes membranaires de cellules d'origines les plus variées. Les caractérisations recherchées s'adressent principalement à des marqueurs de tissu ou d'organe, à des marqueurs d'états de différenciation ou d'activation de cellules normales et à l'identification ou au typage de cellules normales ou cancéreuses. Un domaine d'application particulièrement important est l'étude des lignées cellulaires de l'hématopoïèse (érythrocytaire, megakaryocytaire, granulocytaire, monocytaire, lymphocytaire).

Ainsi, par exemple, les anticorps monoclonaux ont permis de préciser les caractéristiques de surface respectives des lymphocytes T et B. Les marqueurs correspondants seuls ou en combinaison identifient des stades de différenciation et de spécialisation fonctionnelle des lymphocytes. Par convention internationale, les marqueurs de surface des leucocytes humains ont été classés dans des groupes ou classes de différenciation (CD) définis lors du Sous-Comité IUIS-OMS, 1984 et décrits dans Bulletin de l'Organisation Mondiale de la Santé, 1984, *62* (5), 813-815. Au fur et à mesure que les recherches progressent, une proportion croissante de ces antigènes se révèlent être des molécules fonctionnelles et en particulier des récepteurs de ligands spécifiques.

Les récepteurs localisés à la surface des cellules sont détectés classiquement par fixation des ligands radiomarqués correspondants. Ces récepteurs sont fréquemment des récepteurs hormonaux dont les ligands sont des hormones.

Les ligands radiomarqués sont commerciaux ou ils sont préparés selon des méthodes connues (F.C. GREENWOOD, W.M. HUNTER et Coll., Biochem. J., 1963, *89*, 114).

L'immunocapture de cellules sur un support solide est décrite dans la demande de brevet WO 86/02091 dans laquelle le but est d'éliminer des cellules indésirables dans des échantillons de moelle osseuse destinée à des transplantations. Dans cette demande de brevet, la capture des cellules est réalisée sur des microbilles flottantes et nécessite que l'anticorps utilisé soit relié au support solide par une structure macromoléculaire complexe, appelée réseau-relais, capable d'assurer une orientation privilégiée de l'anticorps par rapport à celle de l'antigène cellulaire correspondant. Aucune application de la technique au dosage quantitatif d'un récepteur membranaire n'est indiquée dans cette demande.

L'immunocapture de cellules est également décrite dans la demande de brevet WO 84/03151 pour une application analytique. Dans cette demande, le but est d'identifier les groupes tissulaires auxquels appartiennent les cellules examinées (opération généralement dénommée typage HLA). La capture des cellules est effectuée grâce à des anticorps disposés selon une géométrie particulière sur des supports très spécialisés (lamelles de microscope). Les résultats sont obtenus par simple observation visuelle du support et conduisent à des réponses en "tout ou rien". Ainsi, les systèmes d'immunocapture de cellules décrits jusqu'à ce jour ne conduisent pas à des applications analytiques permettant la détermination quantitative d'un récepteur exprimé à la membrane de certaines cellules.

La méthode de dosage qui fait l'objet de la présente invention, possède des avantages considérables par rapport à toutes les techniques antérieurement connues et utilisées car elle permet de mesurer de façon quantitative tout récepteur membranaire d'une population cellulaire en un seul temps d'analyse. Ce dosage est réalisé sur des cellules qui n'ont subi aucune intervention chimique ou physique et qui sont dans leur état d'intégrité physiologique. De plus, la méthode de dosage selon l'invention possède les caractéristiques de très haute spécificité, propres aux systèmes à double reconnaissance mettant en oeuvre 2 marqueurs différents spécifiques portés par la même cellule l'un étant un antigène choisi pour immunocapturer les cellules que l'on veut analyser, l'autre étant le récepteur membranaire à doser qui est reconnu très spécifiquement par le ligand correspondant. Cette méthode est simple, rapide et reproductible. Elle est tout à fait adaptée à l'analyse d'un grand nombre d'échantillons ce qui permet son utilisation à des fins de diagnostic dans les laboratoires de biologie clinique à grand débit.

Ainsi la présente invention se rapporte à une trousse pour le dosage d'au moins un récepteur membranaire caractéristique d'une population ou sous-population cellulaire comprenant, comme composants:

a) un support solide sur lequel sont fixés par liaison covalente ou adsorption physique un ou plusieurs anticorps monoclonaux dirigés contre des antigènes de surface de la population cellulaire examinée, autres que le dit récepteur, destinés à immobiliser sur le support les cellules parmi lesquelles se trouvent celles de la sous-population portant le récepteur à doser ;

b) une solution contenant, sous forme radiomarquée, le ligand spécifique du récepteur membranaire à doser.

Le terme cellule, utilisé dans la présente description et dans les revendications qui vont suivre, inclut les cellules humaines ou les cellules animales et notamment les cellules du sang, y compris les plaquettes.

La méthode de dosage selon l'invention s'applique à des cellules entières, c'est à dire non lysées, portant le récepteur à doser.

Ces cellules n'ont subi aucune intervention physique ou chimique et elles sont mises en oeuvre dans un état d'intégrité physiologique complète. Cette situation constitue la meilleure garantie d'intégrité des marqueurs membranaires choisis, l'antigène d'immunocapture et le récepteur à doser.

Comme support solide, on peut utiliser tout dispositif adapté à la manipulation de suspensions cellulaires et préférentiellement des tubes, des supports magnétiques particulaires ou des plaques rigides ou souples de microtitration en polyéthylène, polystyrène, chlorure de polyvinyle ou nitrocellulose comportant des micropuits. Les anticorps monoclonaux destinés à l'immobilisation des cellules peuvent être fixés sur les supports solides soit par liaison chimique covalente, soit par adsorption physique selon les techniques classiques bien connues de l'homme de l'art, telles que celles décrites par STOCKER et HEUSSER J. Immunol. Methods, 1979, vol. 26, p. 87-95. Avantageusement, le support peut être au préalable saturé à l'aide d'une protéine.

Selon l'invention, le ou les anticorps monoclonaux fixés sur le support solide doivent permettre l'immunocapture de la population cellulaire parmi laquelle se trouve la ou les populations cellulaires porteuses des récepteurs membranaires à doser. Lorsque cette population est constituée de cellules humaines, les anticorps monoclonaux préférés pour l'immunocapture sont les anticorps anti-HLA de classe I qui sont spécifiques de la partie commune des antigènes HLA A, B et C présents sur les leucocytes et de nombreuses autres lignées cellulaires de l'organisme. Parmi ces anticorps, celui dénommé S-classe I, commercialisé par BYOSIS, est particulièrement préféré.

Dans d'autres cas où les cellules examinées sont des cellules humaines et dans tous les cas lorsque ces cellules ne sont pas humaines, des anticorps monoclonaux appropriés au type de cellules examinées peuvent également être utilisés pour l'immunocapture selon l'invention.

L'expression "un ligand du récepteur sous forme radiomarquée" ou "un ligand marqué par une sonde radioisotopique" signifie que ce ligand porte, sur un élément propre de sa structure, un isotope radioactif permettant de le doser par comptage de la radioactivité qui lui est associée.

La trousse de dosage peut contenir comme composant additionnel une solution tampon destinée au lavage du support solide après immobilisation et marquage des récepteurs membranaires avec le ligand du récepteur radiomarqué.

La trousse de dosage peut aussi contenir comme composants additionnels les échantillons nécessaires à l'étalonnage du dosage ainsi qu'au contrôle de qualité du dosage.

La présente invention a également pour objet un procédé pour le dosage des récepteurs de surface d'une population ou d'une sous-population cellulaire caractérisé en ce qu'il consiste :

a) à immobiliser la population cellulaire portant le récepteur à doser ou une sous-population portant le récepteur à doser, sur un support solide, en utilisant un ou plusieurs anticorps monoclonaux, préalablement fixés par liaison covalente ou par adsorption physique sur ledit support et capable de reconnaître un antigène présent à la surface des cellules autre que le récepteur à doser et, dans la même étape à marquer directement certaines cellules appartenant à la population ou sous-population cellulaire immobilisée, au moyen d'au moins un ligand spécifique de ce récepteur à doser, ledit ligand portant une sonde radioisotopique ;

3

b) à observer une période d'incubation pour permettre l'immunocapture et le marquage simultanés ;

c) à laver le support solide pour éliminer les cellules non immobilisées et l'excès de ligand portant la sonde radioisotopique ;

d) à lire les résultats par comptage de la radioactivité fixée, en se référant éventuellement à une gamme d'étalonnage.

La trousse de dosage et le procédé selon l'invention s'appliquent de façon préférentielle au dosage des récepteurs de surface des éléments figurés du sang humain notamment les leucocytes, plus particulièrement les lymphocytes.

La trousse de dosage et le procédé selon l'invention permettent de mesurer des signaux de radioactivité dépendant à la fois du nombre de cellules présentes dans la population cellulaire examinée et de la densité du récepteur mesuré à la surface de ces cellules. La mesure de ces signaux permet une évaluation quantitative du nombre total de molécules de ce récepteur qui sont portées par la population ou sous-population cellulaire examinée.

A titre d'illustration d'une application de l'invention, on peut citer le cas des lymphocytes T humains, pour lesquels il existe notamment une sous-population de cellules : les lymphocytes caractérisés par la présence du récepteur à l'interleukine 2.

Ce récepteur est exprimé avec une densité très augmentée à la surface des lymphocytes dans le cas où le système immunitaire est sollicité : par exemple, au cours des pathologies infectieuses, dans certaines maladies dysimmunitaires ou dans les greffes d'organes.

En utilisant la trousse de dosage et le procédé selon l'invention, on peut mesurer, sur un échantillon de sang prélevé chez un malade, le nombre de lymphocytes activés et le rapporter éventuellement au nombre des lymphocytes totaux.

De même, on peut mesurer le rapport entre les lymphocytes quiescents et les lymphocytes activés et la variation de ce rapport au cours de l'évolution de la maladie en fonction de la thérapeutique appliquée au malade.

Pour obtenir l'immobilisation spécifique des lymphocytes T de l'échantillon, on utilise de préférence un ou plusieurs anticorps monoclonaux capables seuls ou en combinaison de reconnaître la totalité des cellules T de l'échantillon, ce qui est le cas des anticorps anti-leucocytes communs (ou anti-CD45) ou des anticorps reconnaissant l'ensemble de la population T (dits "pan T") tels que les anticorps anti-CD2 (ou anti-T11), anti-CDS (ou anti-T1), anti-CD7 (ou anti-T2) ou d'autres anticorps pan-T non encore affectés à une classe de différenciation selon les critères OMS.

On peut par exemple, fixer par adsorption un anticorps monoclonal ou un mélange d'anticorps monoclonaux spécifiques de la totalité des antigènes de surface des cellules T sur les parois des micropuits. Ces anticorps monoclonaux permettront d'immobiliser ultérieurement dans les micropuits toute la population des cellules T de l'échantillon examiné. Les plaques ainsi préparées peuvent être lyophilisées et stockées de préférence à 4° C. Cette étape peut être réalisée industriellement et l'on peut ainsi disposer de plaques prêtes à l'emploi pour les trousses de dosage applicables à toute sous-population des lymphocytes T caractériséepar la présence du récepteur de tout ligand identifié qui puisse être obtenu sous forme radiomarquée.

Les échantillons contenant les cellules à doser et provenant du sang ou de tout liquide biologique approprié, normal ou pathologique, peuvent être utilisés tels quels ou après préparation, notamment par centrifugation en gradient de densité selon les méthodes déjà connues et en particulier en milieu à forte densité comme par exemple, le FICOLL-PAQUE, commercialisé par Pharmacia. Pour le dosage des lymphocytes sanguins, on peut également traiter l'échantillon de sang à doser avec une solution appelée tampon de lyse qui lyse les globules rouges.

Des parties aliquotes de la suspension cellulaire appropriée sont placées au contact du support solide, par exemple dans les micropuits d'une plaque de microtitration préalablement préparée, en même temps que la solution faisant partie de la trousse de dosage et contenant le ligand spécifique du récepteur de la population cellulaire visée et portant une sonde radio-isotopique. Les ligands spécifiques des récepteurs membranaires sont généralement accessibles commercialement ; sinon ils sont préparés, par exemple par marquage du ligand à l'iode 125 ou l'iode 131, par exemple en présence de chloramine T selon un procédé connu (F.C. GREENWOOD, W.M. HUNTER et Coll, Biochem. J., 1963, *89*, 114).

La période d'incubation, c'est à dire la durée nécessaire pour l'immobilisation et le marquage simultané des cellules est de préférence inférieure ou égale à 1 heure. Pendant ce délai, le support solide peut éventuellement être centrifugé pour améliorer l'immobilisation des cellules. Le support solide, la plaque de microtitration par exemple, est ensuite lavé pour éliminer en même temps les cellules non fixées et le ligand portant la sonde radio-isotopique, en excès.

La radioactivité associée aux cellules est comptée dans un compteur gamma selon toute modalité appropriée et par exemple après solubilisation des cellules par une solution alcaline (par exemple une solution de soude) et récupération de la solution contenant la radioactivité à l'aide d'un tampon absorbant.

Les résultats du dosage des récepteurs membranaires selon l'invention peuvent être exprimés selon toute modalité appropriée à l'examen effectué. Plus particulièrement on peut exprimer ces résultats en nombre total de molécules d'un récepteur particulier présentes dans un volume donné de l'échantillon examiné (par exemple par microlitre de sang).

Pour déterminer le nombre de molécules d'un récepteur particulier, dans l'échantillon examiné, on utilisera de préférence une gamme d'étalonnage constituée de cellules ou de préparations cellulaires appropriées, portant le récepteur à doser et qui auront été préalablement calibrées par une méthode de référence connue. Ces étalons seront de préférence constitués soit par des cellules identiques dans leur provenance aux cellules qui feront l'objet du dosage, soit par des cellules de lignées cellulaires établies portant le récepteur recherché, soit par des préparations, par exemple de membranes, provenant de ces cellules.

Ces étalons sont alors traités exactement comme les échantillons à examiner. Les signaux qui en découlent servent à construire une gamme d'étalonnage à laquelle sont rapportés les signaux mesurés avec les échantillons à examiner. Les calculs ultérieurs sont classiques.

La méthode de dosage selon l'invention est simple, rapide et reproductible. Son utilisation est tout à fait adaptée à l'analyse d'un grand nombre d'échantillons. Pour en comprendre les avantages par comparaison avec les autres méthodes décrites, il convient d'en analyser les différentes étapes.

L'immobilisation des cellules sur le support solide est la phase du dosage qui présente habituellement le plus de difficultés ou qui est la plus critique dans sa réalisation. Le moyen souvent utilisé est la fixation chimique des cellules par le glutaraldéhyde ou le méthanol dans des cupules traitées ou non à la poly-L-lysine (VAN LEUVEN F. et Coll., J. Immunol. Methods, 1978, 23, 109). Toutefois, les fixations chimiques ainsi mises en oeuvre peuvent diminuer ou même supprimer la détection spécifique recherchée ou au contraire induire des marquages faussement positifs de cellules ce qui est un inconvénient très grave (DROVER et MARSHALL J. Immunol. Methods, 1986, 90, 275-281).

De plus la réalisation du procédé par fixation chimique nécessite plusieurs étapes : la centrifugation des cellules, la préparation du mélange de fixateur, la fixation puis les lavages des cellules fixées.

La dessiccation des cellules à 37°C, suivie ou non d'une fixation au méthanol dans les micropuits a aussi été proposée (BAUMGARTEN, J. Immunol. Methods, 1986, 94, 91-98). En fait, la déshydratation des cellules à 37°C peut altérer certains constituants membranaires fragiles et perméabiliser la membrane plasmique péricellulaire ce qui facilite l'accès du ligand radiomarqué à des structures intracellulaires qui ne sont pas les cibles visées.

De plus, la reproductibilité de ce procédé est incertaine ; en effet, la décantation des cellules dans les micropuits du dosage et la dessiccation des cellules peuvent varier d'une expérience à l'autre. Enfin, ce dosage est long à réaliser car l'étape de dessiccation des cellules nécessite à elle seule un délai supérieur à 2 heures.

L'immobilisation de populations lymphocytaires a été aussi obtenue grâce à l'emploi d'anticorps polyclonaux adsorbés dans des micropuits (STOCKER et HEUSSER J. Immunol. Methods, 1979, 26, 87-95). Cette méthode permet l'immobilisation de cellules étrangères à la seule population que l'on souhaite analyser.

L'utilisation d'anticorps monoclonaux hautement spécifiques et affins adsorbés ou fixés sur le support solide et notamment dans les puits du dosage permet la capture exclusive des cellules recherchées, les autres populations cellulaires non retenues étant éliminées au cours des lavages effectués en fin de marquage des antigènes à doser. De plus, aucun agent chimique ou physique ne modifie les caractéristiques des antigènes à cette étape car les différentes opérations destinées à fixer chimiquement ou physiquement les cellules au support sont supprimées.

Ainsi, selon la présente invention, il a été trouvé que l'immobilisation des cellules par des anticorps monoclonaux est un procédé qui permet de simplifier l'étape d'immobilisation des cellules portant le récepteur à doser, tout en rendant les résultats plus fiables.

Le procédé selon l'invention comportant l'utilisation simultanée, en une seule étape, d'un processus d'immunocapture des cellules entières, sans intervention physique ou chimique sur les cellules, et le marquage de tout ou partie de ces cellules par un ou plusieurs ligands portant directement une sonde radioisotopique, permet, pour la première fois, le dosage quantitatif sur les cellules elles-mêmes de récepteurs membranaires choisis.

Selon l'invention, le marquage direct des cellules immobilisées immunologiquement permet :
- la simplification de la méthode de dosage par suppression des manipulations intermédiaires répétées entre les étapes successives du marquage dans le cas du marquage indirect :

centrifugations des cellules, élimination des réactifs de marquage, remise en suspension ;
- une économie de réactifs ;
- une fiabilité améliorée par diminution du nombre d'étapes et de manipulations;
- un gain de temps;
- la possibilité de traiter en même temps, avec l'emploi exclusif de matériels et d'appareillages usuels, de grands nombres d'échantillons.

La période d'incubation pour l'immobilisation de la population cellulaire et simultanément le marquage direct des récepteurs de la sous-population cellulaire à doser est courte. Elle est inférieure ou égale à 1 heure dans le cas du dosage des lymphocytes T activés portant le récepteur de l'interleukine-2.

Après le lavage du support solide, le dosage proprement dit est effectué par l'observation d'un signal précis et simple à mesurer avec des appareillages usuels : la radioactivité.

Ainsi l'ensemble du procédé selon l'invention présente de nombreux avantages : il est rapide, fiable, économique et simple.

Le procédé selon l'invention permet de doser des récepteurs de surface d'une population cellulaire dans une large gamme de concentrations cellulaires.

La sensibilité de la méthode vis à vis du nombre de cellules dépend de la densité de récepteur de la population cellulaire dosée. Pour chaque récepteur, on peut, si on le souhaite, définir la concentration minimale molaire en récepteur pouvant être mesurée par le procédé selon l'invention.

On a vérifié que les signaux enregistrés (comptage de radioactivité) permettent d'obtenir des courbes d'étalonnage régulières et satisfaisantes en fonction du nombre de cellules mises en oeuvre, dans les conditions usuelles de manipulation.

Dans les exemples qui vont suivre, on utilisera indifféremment les termes suivants ou leurs abréviations :

BSA : albumine de sérum bovin, ou sérum albumine bovine
PBS : tampon phosphate salin à pH 7,4
cpm : coups par minute
dpm : désintégrations par minute

## EXEMPLE 1

### DOSAGE DES RECEPTEURS DE L'INTERLEUKINE 2 AU MOYEN DU LIGAND PHYSIOLOGIQUE MARQUE.

Les récepteurs de l'interleukine 2 apparaissent sur la membrane des lymphocytes T activés lorsque le système immunitaire est sollicité.

Au laboratoire, on peut travailler sur un échantillon de sang non pathologique, à condition d'obtenir des lymphocytes activés à partir des lymphocytes quiescents par intervention d'un agent exogène tel que la phytohémagglutinine (PHA), la concanavaline A ou d'autres lectines. Le dosage des récepteurs de l'interleukine 2 à partir de sang humain utilise l'interleukine 2 marquée à l'iode 125 accessible commercialement (NEX-229, DUPONT).

A) Séparation des lymphocytes du sang total et activation à la PHA.

On prélève 0,5 ml de l'échantillon de sang à doser que l'on mélange à 1,5 ml de tampon phosphate salin. Dans un tube à hémolyse de 5 ml, on introduit 1,5 ml de Ficoll-Paque, commercialisé par Pharmacia et l'on dépose à la surface de la couche de Ficoll l'échantillon de sang dilué dans le PBS. Après 5 minutes de centrifugation à 900 x g, à température ambiante, on prélève stérilement l'anneau de suspension contenant les cellules mononucléées sous un volume de 0,5 ml. Cet échantillon est additionné de 1,5 ml de milieu de culture. Les cellules sont ensuite dénombrées puis mises en culture à raison de $5.10^5$ cellules par ml de milieu RPMI 1640 (Flow) contenant 10 % de sérum de veau foetal (Flow).

L'activation des lymphocytes T est obtenue avec la PHA (HA 15. WELLCOME) introduite à la concentration finale de 50 $\mu$g/ml de milieu de culture, pendant 3 jours.

B) Préparation de la plaque.

On utilise une plaque de microtitration en plastique commercialisée par NUNC (Référence 64394) comportant 96 micropuits. On place par micropuits 200 μl d'une solution contenant l'anticorps monoclonal purifié anti-CD2 (dénommé ST 11) utilisé pour immobiliser les lymphocytes T totaux, c'est-à-dire pour effectuer leur immunocapture. Cet anticorps commercialisé par BIOSYS, Compiègne, France, sous la référence ST 11 est utilisé à la concentration de 10 μg/ml dans un tampon phosphate salin à pH 7,4 (PBS).

L'adsorption de l'anticorps monoclonal s'effectue à 4°C pendant 12 heures. On élimine l'anticorps en excès par retournement de la plaque.

On prépare une solution contenant 0,1 % de gélatine et 0,5 % de BSA dans un tampon phosphate salin. On introduit 250 μl de cette solution par micropuits, afin de saturer la surface des puits en protéine, ce qui est obtenu après 1 heure à 37°C ; on lave les plaques 3 fois a l'aide de tampon phosphate salin. Les plaques ainsi préparées sont lyophilisées et stockées à 4°C dans un sachet plastique scellé.

C) Incubation des cellules.

Le dosage est réalisé en introduisant dans les micropuits de la plaque un volume de 100 μl de suspension cellulaire (2,5 $10^5$ cellules par ml) contenant les cellules traitées à la PHA pendant 3 jours. On ajoute ensuite 100 μl d'une solution d'interleukine 2 marquée à l'iode 125 (produit réf. NEX-229 DUPONT soit 120 000 dpm dans 100 μl de tampon PBS contenant 5 % de sérum de veau). Après 1 heure à la température ambiante, les micropuits sont vidés par retournement de la plaque. On lave les micropuits 4 fois avec 200 μl de PBS par puits. On introduit ensuite 75 μl de solution d'hydroxyde de sodium 1 M dans chaque puits. Après 10 minutes, le contenu de chaque puits est récupéré avec un tampon absorbant avant comptage de la radioactivité au compteur γ (compteur multipuits LKB).

Les résultats sont calculés et exprimés en dpm, tableau ci-dessous.

|  | Nombre de cellules de l'échantillon 2,5 X $10^5$ cellules | Blanc sans cellule |
|---|---|---|
| Récepteur à l'interleukine 2 | 435 dpm | 185 dpm |

## Revendications

1. Trousse pour le dosage d'un récepteur de surface caractéristique d'une population ou d'une sous-population cellulaire comprenant, comme composants :

a) un support solide sur lequel sont fixés par liaison covalente ou par adsorption physique un ou plusieurs anticorps monoclonaux dirigés contre des antigènes de surface de la population cellulaire à doser autres que le dit récepteur ;

b) une solution contenant, le ligand spécifique du récepteur membranaire à doser, le dit ligand portant une sonde radioisotopique ;

c) éventuellement un composant additionnel constitué par une solution tampon de lavage et/ou des échantillons permettant l'étalonnage et le contrôle de qualité du dosage.

2. Trousse selon la revendication 1, dans laquelle le composant (a) est un support solide constitué par une plaque de microtitration dans les puits de laquelle sont fixés le ou les anticorps destinés à immobiliser les cellules parmi lesquelles se trouvent celles de la population ou sous-population portant le récepteur à doser.

3. Trousse selon la revendication 1, dans laquelle le composé (a) est un support magnétique particulaire.

4. Trousse selon l'une quelconque des revendications 1 à 3 dans laquelle le composant (a) est constitué d'un support solide sur lequel sont fixés un ou plusieurs anticorps monoclonaux HLA de classe I.

5. Trousse selon la revendication 4, dans laquelle l'anticorps monoclonal fixé est l'anticorps dénommé S-classe I.

6. Trousse selon l'une des revendications 1 à 5, dans laquelle le ligand spécifique est l'interleukine 2 marquée à l'iode 125.

7. Procédé de dosage des récepteurs de surface d'une population ou sous-population cellulaire, caractérisé en ce qu'il consiste :

a) à immobiliser la population cellulaire portant le récepteur à doser ou une population cellulaire comprenant la sous-population portant le récepteur à doser sur un support solide, en utilisant un ou plusieurs anticorps monoclonaux, préalablement fixés par liaison covalente ou par adsorption physique sur ledit support et capable de reconnaître un antigène présent à la surface des cellules autre que le récepteur à doser,

et, dans la même étape, à marquer directement certaines cellules de la population ou sous-population cellulaire immobilisée au moyen d'au moins un ligand spécifique du récepteur membranaire à doser, ledit ligand portant une sonde radioisotopique ;

b) à observer une période d'incubation ;

c) à laver le support pour éliminer les cellules non immobilisées et l'excès du ligand ;

d) à lire les résultats par comptage de la radio-activité en se référant éventuellement à une gamme d'étalonnage.

8. Procédé de dosage selon la revendication 7, dans lequel le support solide est tel que défini dans les revendications 2 ou 3.

9. Procédé de dosage selon la revendication 7, dans lequel les ligands portent une sonde radio-isotopique à l'iode 125 ou à l'iode 131.

10. Procédé selon l'une quelconque des revendications 7, 8 ou 9, dans lequel la durée totale d'exécution du dosage est inférieure ou égale à 1 heure.

11. Procédé selon la revendication 7, dans lequel le ou les anticorps monoclonaux fixés sur le support solide sont des anticorps anti-HLA de classe I.

12. Procédé selon la revendication 7, dans lequel l'anticorps monoclonal fixé sur le support solide est l'anticorps dénommé S-classe I.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | WO-A-8 808 538 (TANOX BIOSYSTEMS, INC.) --- | | G 01 N 33/577 G 01 N 33/543 |
| A | EP-A-0 054 676 (BOEHRINGER MANNHEIM GmbH) --- | | |
| A | EP-A-0 224 134 (BOEHRINGER MANNHEIM GmbH) --- | | |
| A | US-A-4 770 995 (B.Y. RUBIN et al.) --- | | |
| A | BIOLOGICAL ABSTRACTS, vol. 78, no. 8, 1984, résumé no. 57796, Philadelphia, PA, US; F.H. SARKAR et al.: "Interferon receptor interaction: Internalization of interferon alpha2 and modulation of its receptor on human cells", & EUR. J. BIOCHEM. 140(3): 461-468. 1984 --- | | |
| A | WO-A-8 806 918 (COULTER ELECTRONICS, INC.) --- | | **DOMAINES TECHNIQUES RECHERCHES (Int. Cl.5)** |
| A | EP-A-0 179 007 (IMMUNOTECH) & WO-A-86 02 091 (Cat. D) --- | | G 01 N |
| D,A | WO-A-8 403 151 (CENTOCOR INC.) ----- | | |

Le présent rapport a été établi pour toutes les revendications

| Lieu de la recherche | Date d'achèvement de la recherche | Examinateur |
|---|---|---|
| LA HAYE | 25-06-1990 | GRIFFITH G. |